# EUROPÄISCHE PATENTSCHRIFT

(11) **EP 1 786 572 B1**
(45) Veröffentlichungstag und Bekanntmachung des Hinweises auf die Patenterteilung: **11.03.2009**
(21) Anmeldenummer: 05782590.3
(22) Anmeldetag: 12.09.2005
(51) Int. Cl.: B05D 5/00, B05D 7/00

(54) **OBERFLÄCHENSTRUKTURIERTE SUBSTRATE UND IHRE HERSTELLUNG**
SURFACE-STRUCTURED SUBSTRATE AND PRODUCTION THEREOF
SUBSTRATS A SURFACE STRUCTUREE ET PRODUCTION DESDITS SUBSTRATS

(30) Priorität: 10.09.2004 DE 102004043908
(43) Veröffentlichungstag der Anmeldung: 23.05.2007
(73) Patentinhaber: Max-Planck-Gesellschaft zur Förderung der Wissenschaften e.V., 80539 München (DE)
(72) Erfinder: GRÄTER, Stefan, 73732 Esslingen (DE)
(74) Vertreter: Hertz, Oliver
(86) Internationale Anmeldenummer: PCT/EP2005/009787
(87) Internationale Veröffentlichungsnummer: WO 2006/027274

(56) Entgegenhaltungen:
- WO-A-99/21652
- DE-A1- 19 747 815
- DE-A1- 19 843 411
- US-A1- 2002 123 227

## Beschreibung

Die vorliegende Erfindung betrifft oberflächenstrukturierte Substrate, die geordnete Oberflächenstrukturen im Nanometerbereich aufweisen, sowie ein Verfahren zur Herstellung dieser Substrate mit Oberflächenstrukturierung im Nanometerbereich.

Periodisch und aperiodisch mikrostrukturierte Oberflächen von einigen Mikrometern bis einigen Nanometern werden für eine Vielzahl von Anwendungen, insbesondere elektronische und optische Bauelemente, sowie Sensoren und in der Mikrotechnologie verwendet. Die Herstellung solcher mikrostrukturierter Oberflächen erfolgt durch Anwendung bekannter lithographischer Techniken, die je nach Art der gewünschten Mikrostruktur geeignet ausgewählt werden. So können beispielsweise mit der Elektronenstrahl- und Ionenstrahllithographie Strukturen im Nanometerbereich hergestellt werden, und entsprechende Anlagen sind im Handel erhältlich. Ferner erlaubt die Atomstrahllithographie durch die Kontrolle der Wechselwirkungen der Atomstrahlen mit Lichtmasken, dass großflächige periodische Linienmuster und verschiedene zweidimensionale periodische Strukturen erzeugt werden.

Da diese Verfahren jedoch den Nachteil aufweisen, dass sie ökonomisch nicht vertretbar sind und/oder keine periodischen Strukturen im Nanometerbereich liefern und/oder nur durch physikalische Parameter gesteuert werden können und deshalb apparativ sehr aufwendig sind, wurde die sogenannte mizellare Blockcopolymer-Nanolithographie entwickelt, mit der sich nanostrukturierte Oberflächen mit einer Periodizität im unteren Nanometerbereich zwischen 10 und 170 nm herstellen lassen. Das mizellare Blockcoplymer-Nanolithographie-Verfahren ist im Detail in den folgenden Patenten und Patentanmeldungen beschrieben: DE 199 52 018, DE 197 47 813, DE 297 47 815 und DE 197 47 816.

Bei der mizellaren Blockcopolymer-Nanolithographie spielen Templateffekte eine wichtige Rolle. Darunter versteht man die Vorgabe von Hilfsstrukturen, welche das Wachstum, die Struktur und die Anordnung des darauf aufbauenden Systems kontrolliert. Solche Template sind beispielsweise Blockcopolymere und Pfropfcopolymere, die in geeigneten Lösungsmitteln zu mizellaren Kern-Schalesystemen assoziieren, sowie hochverzweigte dendritische Moleküle mit einer Kern-Schale-Struktur. Diese Kern-Schale-Strukturen dienen zur Lokalisierung anorganischer Vorstufen, aus denen nanometergroße, anorganische Teilchen mit einer kontrollierten Größe, durch die Polymerhülle räumlich voneinander getrennt, abgeschieden werden können. Vorteilhaft ist dabei insbesondere, dass die Kern-Schale-Systeme oder Mizellen durch einfache Abscheidungsvorgänge wie Spin-Casting oder Dip-Coating als hochgeordnete Monofilme auf unterschiedliche Substrate aufgebracht werden können. Die organische Matrix wird anschließend z.B. durch einen Gas-Plasma-Prozess oder durch Pyrolyse rückstandsfrei-entfernt, wodurch anorganische Nanopartikel in der Anordnung auf dem Substrat fixiert werden, in der sie durch das organische Templat positioniert wurden. Die Größe der anorganischen Nanopartikel wird durch die Einwaage einer bestimmten anorganischen Precursorverbindung und der laterale Abstand zwischen den Nanopartikeln durch die Struktur, insbesondere durch das Molekulargewicht der organischen Matrix bestimmt. Dadurch konnten Teilchengrößen von Au-, Ag-, Pt-, Pd-, Ni-, Co-, Fe- und Ti-Teilchen sowie deren Oxide und Legierungen zwischen 1 und 20 nm in geordneten Mustern auf Substraten abgeschieden werden, wobei die Muster eine Periodizität entsprechend dem sphärischen Kern-Schale System zwischen 10 und 170 nm aufwiesen.

In der internationalen Patentanmeldung WO-A-99/21652 wird ein Verfahren zur Herstellung einer strukturierten oder dekorierten Oberfläche beschrieben, bei dem ein mit Metallclustern beladenes Kern-Schale-Polymersystem auf ein Substrat aufgebracht und anschließend das Polymer durch z.B. ein Ionenätzverfahren oder nasschemisches Ätzverfahren entfernt wird, wodurch die Metallcluster mit geordneter Struktur auf dem Substrat zurückbleiben. Bei diesem Verfahren wird keine von einem ersten Substrat vorgegebene Cluster-Struktur auf ein zweites Substrat übertragen.

Voraussetzung für das oben beschriebene mizellare Blockcopolymer-Nanolithographie-Verfahren ist, dass die Substrate aus Materialien bzw. Materialmischungen bestehen müssen, die den Gas-Plasma- oder Pyrolyse-Prozess oder andere Ätzverfahren zur Entfernung der organischen Matrix unbeschadet überstehen. Als Substrate werden daher üblicherweise Edelmetalle, oxidische Gläser, mono- oder multikristalline Substrate, Halbleiter, Metalle mit oder ohne passivierter Oberfläche, Isolatoren oder allgemein Substrate mit hoher Resistenz gegen nachfolgende Ätzprozeduren eingesetzt. Organische Substrate und eine Vielzahl anorganischer Substrate scheiden jedoch augrund ihrer Unbeständigkeit gegenüber dem Gas-Plasma- oder Pyrolyse-Prozess und anderen Ätzverfahren für die Anwendung in dem Blockcopolymer-Nanolithographie-Verfahren aus. Außerdem sind Substrate ausgeschlossen, deren Oberfläche nicht ebenmäßig genug sind, um eine regelmäßige Selbstorganisation der Polymermizellen zuzulassen. Auch die Beschichtung von Membranen, die wenige Nanometer dick sind, lässt sich mit diesem Verfahren technisch nicht realisieren.

Dies ist insofern nachteilig, als insbesondere sowohl organische Polymersubstrate als auch die für das Blockcopolymer-Nanolithographie-Verfahren nicht einsetzbaren anorganischen Substrate für die Herstellung von beispielsweise Leiterbahnen bei der Chip-Herstellung, bei der Kultivierung von Zellen, Bakterien und Viren sowie für den Einsatz als Implantate eine große praktische und wirtschaftliche Bedeutung haben.

Beispielsweise werden Arbeiten mit einer großen Anzahl an Zellen und Zellkulturen aufgrund praktischer und ökonomischer Gesichtspunkte mit Kulturschalen durchgeführt, die aus Plastik oder besonderen Polymeren bestehen. Diese werden beispielsweise zur Vermehrung von Zellen, zur Differenzierung von Zellen oder zur Generierung von Gewebe im Allgemeinen eingesetzt. Die Nanostruktur konnte jedoch bisher nicht auf polymere Oberflächen übertragen und somit bisher nicht zur Einstellung von adhäsionsvermittelter Zellfunktion genutzt werden.

Ferner hat die Anwendung der bekannten Substrate aus Metallen, Gläsern, mono- oder multikristallinen Substraten und Halbleitern den Nachteil, dass sie eine hohe und nicht beliebig einstellbare Festigkeit aufweisen. Es besteht jedoch die Nachfrage nach strukturierten Oberflächen, die weich und flexibel sind und sich z.B. in Form einer Folie auf Gegenstände, wie Implantat- oder Stent-Materialien, aufbringen lassen und diese Gegenstände so mit einer strukturierten Oberfläche versehen können. Außerdem spielt die Festigkeit der Oberflächen für die Differenzierung von darauf wachsendes Zellen eine Rolle. Zudem ist bei einer Vielzahl von Anwendungen in der Biologie, Optik, Sensorik und Elektronik die chemische Beschaffenheit der Oberfläche zwischen den erzeugten Strukturen, die bisher stark eingeschränkt war, von großer Bedeutung.

Daher besteht die der vorliegenden Erfindung zugrundeliegende Aufgabe darin, ein Verfahren zur Nanostrukturierung von Substratoberflächen bereitzustellen, die bisher nicht oder nur sehr schwierig strukturiert werden konnten.

Diese Aufgabe wird gelöst durch ein Verfahren zur Herstellung eines oberflächenstrukturierten Substrats, umfassend die Schritte:
(i) Herstellen eines ersten Substrats, das auf mindestens einer Oberfläche mit anorganischen Nanoclustern nanostrukturiert ist,
(ii) Aufbringen eines Substratmaterials für ein zweites Substrat, das vom ersten Substratmaterial verschieden ist, auf die im Schritt (i) erhaltene nanostrukturierte Oberfläche des ersten Substrats, und
(iii) Abtrennen des ersten Substrats von dem zweiten Substrat aus Schritt (ii), einschließlich der anorganischen Nanocluster, wodurch ein mit Nanoclustern nanostrukturiertes zweites Substrat erhalten wird.

Gemäß einer bevorzugten Ausführungsform wird in dem obigen Schritt (ii) für das zweite Substratmaterial ein härtbares Substratmaterial eingesetzt, wobei das härtbare Substratmaterial ausgewählt ist aus einem organischen vernetzbaren oder nichtvernetzbaren Polymer, einem Harz, einem organischen polymerisierbaren und/oder vernetzbaren Oligomer und einem organischen polymerisierbaren Polymerprecursor oder Mischungen davon, und gehärtet wird. Diese bevorzugte Ausführungsform ist dementsprechend ein Verfahren zur Herstellung eines polymeren oberflächenstrukturierten Substrats, umfassend die Schritte:
(a) Herstellen eines ersten Substrats, das auf mindestens einer Oberfläche mit anorganischen Nanoclustern nanostrukturiert ist,
(b) Aufbringen eines härtbaren Substratmaterials für ein zweites Substrat, das vom ersten Substratmaterial verschieden ist, auf die im Schritt (a) erhaltene nanostrukturierte Oberfläche des ersten Substrats, wobei das härtbare Substratmaterial ausgewählt ist aus einem organischen vernetzbaren oder nichtvernetzbaren Polymer, einem Harz, einem organischen polymerisierbaren und/oder vernetzbare Oligomer und einem organischen polymerisierbaren Polymerprecursor oder Mischungen davon,
(c) Härten des Substratmaterials für das zweite Substrat und
(d) Abtrennen des ersten Substrats von dem in Schritt (c) erhaltenen zweiten Substrat und den anorganischen Nanoclustern, wodurch ein mit Nanoclustern nanostrukturiertes zweites Substrat erhalten wird.

Gemäß einer anderen bevorzugten Ausführungsform werden die in Schritt (ii) verwendbaren zweiten Substratmaterialien durch thermische Verdampfung, Elektronenstrahlverdampfung, Sputtern oder elektrochemisches Abscheiden auf das erste Substrat aufgebracht.

Im folgenden werden die einzelnen Schritte des Verfahrens zur Herstellung eines oberflächenstrukturierten Substrats der vorliegenden Erfindung näher erläutert.

In Schritt (i) bzw. (a) wird eine mit anorganischen Nanoclustern nanostrukturierte Oberfläche hergestellt.

Als Verfahren zur Herstellung solcher nanostrukturierten Oberflächen können die im Stand der Technik bekannten Lithographie-Verfahren angewendet werden. Bevorzugt ist die Herstellung durch das mizellare Blockcopolymer-Nanolithographie-Verfahren, wodurch nanostrukturierte Oberflächen auf einfache und kostengünstige Weise hergestellt werden können (s. DE 199 52 018, DE 197 47 813, DE 297 47 815 und DE 197 47 816).

Wenn die nanostrukturierte Oberfläche durch ein im Stand der Technik bekanntes Lithographie-Verfahren hergestellt wird, besteht das verwendete erste Substrat aus einem Material, das üblicherweise in diesen Lithographie-Verfahren eingesetzt wird. Im Fall des mizellaren Blockcopolymer-Nanolithographie-Verfahrens sind als verwendbare Substratmaterialien insbesondere zu nennen: Edelmetalle, oxidische Gläser, mono- oder multikristalline Substrate, Halbleiter, Metalle mit oder ohne passivierte Oberfläche, Isolatoren oder allgemein Substrate mit hoher Resistenz gegen nachfolgende Ätzprozeduren. Vorzugsweise handelt es sich hierbei um Pt, Au, GaAs, AlₓGaAs, Si, SiO₂, Ge, SiₓN_{y}, SiₓGaAs, InP, InPSi, GaInAsP, Glas, Graphit, Diamant, Glimmer, SrTiO₃ sowie deren dotierte Modifikationen.

Es ist auch möglich, dass das erste Substrat aus einem polymeren Material besteht. Ein solches nanostrukturiertes polymeres Substrat ist erhältlich durch das hier beschriebene Verfahren der vorliegenden Erfindung. Das in dem Verfahren der vorliegenden Anmeldung in Schritt (d) erhältliche Substrat kann somit selbst wiederum als ein erstes Substrat in Schritt (i) bzw. (a) eingesetzt werden. Als polymeres Material sind jegliche Polymere verwendbar. Als besonders gut geeignete polymere Materialien können beispielhaft genannt werden: Polystyrol, Epoxidharze, Polydimethylsiloxan (PDMS) und Polyethylenglycoldiacrylate (PEG-DA). Besonders bevorzugt ist ein erstes Substrat aus Polystyrol.

In der obigen Ausführungsform, bei der die in Schritt (ii) verwendbaren zweiten Substratmaterialien durch thermische Verdampfung, Elektronenstrahlverdampfung, Sputtern oder elektrolytisches Abscheiden auf das erste Substrat aufgebracht werden, wird bevorzugt ein erstes Substrat aus einem polymeren Material, wie oben angegeben, verwendet.

Die in Schritt (i) bzw. (a) hergestellten anorganischen Nanocluster sind insbesondere sauerstoffresistente Edelmetalle, wie Au, Pt, Pd oder Oxide, wie z.B. halbleitende Oxide wie TiO₂, oder magnetische Teilchen wie z.B. bestimmte Modifikationen des Fe₂O₃. Ferner sind auch Cluster aus metallischen Mischsystemen, wie Au/Fe₂O₃, AuCoO, Au/Co₃O₄, Au/ZnO, Au/TiO₂, Au/ZrO₂, Au/Al₂O₃, Au/In₂O₃, Pd/Al₂O₃, Pd/ZrO₂, Pt/Al₂O₃ und Pt/Graphit, denkbar. Bevorzugt sind die anorganischen Nanocluster aus Au.

Die in Schritt (i) bzw. (a) auf das erste Substrat aufgebrachten Cluster sind hinsichtlich ihrer Form nicht beschränkt und können als Punkte, Linien, Flächen oder andere beliebige Formen vorliegen. Die Cluster besitzen vorzugsweise eine Größe von 1 nm bis 300 µm, besonders bevorzugt 1 nm bis 150 µm und noch bevorzugter 1 nm bis 20 µm. Im Fall von punktförmigen Clustern ist mit "Größe der Cluster" der Durchmesser gemeint. Im Fall von Linien ist mit "Größe der Cluster" die Linienbreite gemeint, wobei die Länge der Linien beliebig ist.'Im Fall von Cluster-Flächen ist die "Cluster-Größe" ein Maß für die Fläche des Clusters.

Die erhaltenen Strukturen, die auf dem ersten Substrat erzeugt werden, weisen bevorzugt Größenordnungen von 1 nm bis 300 µm auf, im Fall von geordneten Strukturen weisen sie Periodizitäten 1 nm bis 300 µm. Im Hinblick auf eine Zelladhäsion auf einem oberflächenstrukturierten Substrat ist eine Größenordnung bzw. Periodizität von 5 nm bis 100 µm besonders bevorzugt. Noch bevorzugter ist eine Größenordnung bzw. Periodizität von 5 nm bis 100 nm und ganz besonders bevorzugt 5 nm bis 20 nm. Eine Größenordnung von 5 bis 500 nm lässt sich beispielsweise durch das mizellare Blockcopolymer-Nanolithographie-Verfahren herstellen. Größere Strukturen lassen sich z.B. durch bekannte photolithographische Verfahren erzeugen.

In dem Schritt (b) des Verfahrens zur Herstellung eines polymeren oberflächenstrukturierten Substrats der vorliegenden Erfindung wird ein härtbares Material für ein zweites Substrat auf die in Schritt (a) erhaltene strukturierte Oberfläche des ersten Substrats aufgebracht. Das härtbare Material für das zweite Substrat ist ausgewählt aus einem organischen oder anorganischen Polymer, einem Harz, einem organischen polymerisierbaren oder vernetzbaren Oligomer, einem vernetzbaren Polymer und einem organischen polymerisierbaren Polymerprecursor. Als spezielle Beispiele für das härtbare zweite Substratmaterial können Polystyrol, Epoxidharze, Polydimethylsiloxan (PDMS), Polyethylenglycoldiacrylate (PEG-DA), z.B. PEG-DA 500, PEG-DA 4000 und PEG-DA 8000, und Polyphosphazene unterschiedlichster Molekulargewichte genannt werden.

Eine spezielle Ausführungsform sieht Kollagene, Hyaluronsäure, Fibronektin, Vitronektin und weitere natürliche Polymere als härtbares Polymer, das in Schritt (b) verwendbar ist, vor.

Für elektronische Anwendungen sind organische und anorganische leitende und halbleitende Polymere von besonderem Interesse, z.B. Poly(4,4-dioctylcyclopentadithiophene).

Das härtbare Material für das zweite Substrat wird vorzugsweise in Form einer Flüssigkeit oder als Lösung durch ein im Stand der Technik übliches Beschichtungsverfahren gleichmäßig mit der gewünschten Dicke aufgetragen. Beispielsweise werden Polystyrol, Poly(4,4-dioctylcyclopentadithiophene) und Polyphosphazene in Lösung und PDMS und Epoxidharze in flüssigem Zustand durch ein Spinschleuder-Verfahren auf das erste Trägermaterial aufgetragen. PEG-Diacrylat wird vorzugsweise in Lösung unter Schutzgas aufgetropft.

Es ist auch möglich, das zweite Substrat aus Schritt (b) auf der Seite, die dem ersten Substrat gegenüberliegt, zusätzlich mit einer anorganischen Trägerschicht zu versehen. Die anorganische Trägerschicht kann aus jeglichem anorganischen Material hergestellt werden, bevorzugt aus einem leitenden anorganischen Material, und besteht beispielsweise aus Silizium, Zinkoxid, Gold, Kohlenstoff. Die anorganische Trägerschicht kann abhängig vom anorganischen Material durch bekannte Verfahren, wie z.B. durch thermische Verdampfung, Elektronenstrahlverdampfung, Sputtern, elektrochemisches Abscheiden oder als Feststoff mit glatter Oberfläche auf das zweite Substrat aufgebracht werden. In dieser Ausführungsform ist es bevorzugt, dass das zweite Substrat nach dem Härten in Schritt (c) lediglich eine Dicke von 1 bis 20 nm aufweist. Dadurch kann erreicht werden, dass die auf dem ersten Substrat aufgebrachten Nanocluster, z.B. Goldcluster, so in das zweite Substrat eingebettet werden, dass sie gleichzeitig mit der anorganischen Trägerschicht Kontakt haben und dadurch zur Herstellung von Nanoelektroden geeignet sind.

In Schritt (c) wird das härtbare Material für das zweite Substrat gehärtet. Das Verfahren zum Härten des zweiten Substratmaterials wird abhängig von der Natur des Substratmaterials geeignet ausgewählt. So werden beispielsweise Polystyrol und Polyphosphazene, die als Lösungen aufgebracht werden können, durch langsames Evaporieren des Lösungsmittels gehärtet. PDMS und Epoxidharze werden thermisch polymerisiert bzw. vernetzt und dadurch ausgehärtet. PEG-Diacrylate werden photochemisch polymerisiert und auf diese Weise gehärtet.

Nach dem Härten weist das zweite Substrat vorzugsweise eine Dicke von 1 nm bis einigen cm auf, insbesondere bevorzugt ist eine Dicke von 10 nm bis 100 cm. Im Hinblick auf eine Anwendung des polymeren oberflächenstrukturierten Substrats als Folie weist das zweite Substrat nach dem Schritt (c) vorzugsweise eine Dicke von 10 nm bis 1 cm auf, besonders bevorzugt 100 nm bis 1 mm.

In der Ausführungsform, bei der die in Schritt (ii) verwendbaren zweiten Substratmaterialien durch thermische Verdampfung, Elektronenstrahlverdampfung, Sputtern oder elektrochemisches Abscheiden auf das erste Substrat aufgebracht werden, sind diese verwendbaren zweiten Substratmaterialien Leiter oder Halbleiter, bevorzugt ausgewählt aus Si, C, Zinkoxid, Cr, Indiumoxid, Cu, Indiumarsenid, Galliumarsenid und Hexadecafluorphthalocyanin (F16CuPc), oder Materialien, die für optisch relevante Beschichtungen verwendbar sind, wie Aluminiumoxid, Calciumfluorid und Magnesiumfluorid. Beispielsweise lässt sich eine F16CuPc-Schicht unter Verwendung eines Mikowellengenerators bei folgenden Bedingungen aufbringen: Temperatur: 125°C, Druck: 2x6⁻⁵ Torr, Behandlungsdauer: 100 sec. Eine ca. 50 nm dicke Cr-Schicht kann beispielsweise durch Sputtern erhalten werden. Dazu wird mit einer Beschleunigungsspannung von 60 mA und einem Argondruck von 1x10⁻⁵ Torr gearbeitet. Wie im Fall des oben beschriebenen härtbaren zweiten Substratmaterials weisen die so hergestellten Schichten ebenfalls bevorzugt eine Dicke von 10 nm bis 1 cm, besonders bevorzugt 100 nm bis 1 mm, auf.

Im Schritt (iii) bzw. (d) des Verfahrens zur Herstellung eines oberflächenstrukturierten Substrats bzw. polymeren oberflächenstrukturierten Substrats wird das erste Substrat von dem in Schritt (ii) aufgebrachten zweiten Substrat bzw. von dem in Schritt (c) gehärteten zweiten Substrat, einschließlich der Nanocluster, abgetrennt, wodurch ein mit anorganischen Nanoclustern im Nanometerbereich strukturiertes zweites Substrat erhalten wird, wobei die Nanocluster mit dem gleichen Muster auf das zweite Substrat übertragen werden, das die Nanocluster auf dem ersten Substrat aufwiesen. Die Abtrennung erfolgt in einem geeigneten Verfahren entweder mechanisch oder durch ein Abtrennmittel, das abhängig von der Natur des ersten Substratmaterials und unter Berücksichtigung der Natur des zweiten Substratmaterials geeignet ausgewählt wird. Zu beachten ist dabei, dass das eingesetzte Abtrennmittel so ausgewählt wird, dass zwar das erste Substrat von dem Abtrennmittel angegriffen wird, beispielsweise durch Lösen, aber umgekehrt das zweite Substrat gegenüber diesem Abtrennmittel so stabil und unempfindlich wie möglich ist. So lässt sich beispielsweise ein erstes Substrat, das aus Glas oder Siliziumdioxid besteht, mit Flusssäure (z.B. 25%ig) entfernen, wenn das zweite Substrat aus Polystyrol, Epoxidharz, Polydimethylsiloxan (PDMS), Polyethylenglycol-diacrylat (PEG-DA) oder Phosphazen besteht. Im Fall von Polystyrol als erstes Substrat kann beispielsweise Toluol als geeignetes Abtrennmittel eingesetzt werden, wenn das zweite Substrat aus PEG-DA oder PDMS besteht.

Die Größe des in Schritt (iii) bzw. (d) erhaltenen Substrats bzw. polymeren Substrats mit strukturierter Oberfläche ist beliebig und kann je nach gewünschter Anwendung zwischen 100 nm² und mehreren Metern betragen. Bevorzugt ist eine Größe von 1 mm² bis 100 cm².

Gemäß einer bevorzugten Ausführungsform des Verfahrens der vorliegenden Erfindung kann zwischen Schritt (i) und Schritt (ii) bzw. Schritt (a) und Schritt (b) ein Schritt der Immobilisierung eines Anbindungsmoleküls durchgeführt, vorzugsweise ausgewählt aus Propenthiol, Mercaptopolyethylenglykolacrylat, Cysteamin mit Acryloylchlorid, Polyethylenglykoldithiol, Alkylthioglykolat und Amino-1-alkylthiol. "Alkyl" bezeichnet einen geraden oder verzweigten, gesättigten oder ungesättigten Kohlenwasserstoff mit 1 bis 24 Kohlenstoffatomen, bevorzugt 4 bis 18 Kohlenstoffatome, besonders bevorzugt 6 bis 12 Kohlenstoffatome. Als besonders vorteilhaft haben sich Amino-1-undecanthiol und Allyl-mercaptoacetat gezeigt. Das Anbindungsmolekül sollte derart ausgewählt werden, dass es sich spezifisch an die anorganischen Nanocluster anbinden lässt und sich chemisch oder elektrostatisch mit dem zweiten Substrat verbindet, aber an dem ersten Substrat nicht haftet.

Die Immobilisierung der Anbindungsmoleküle erfolgt durch bekannte Verfahren. Beispielsweise kann die Anbindung von Propenthiol an Goldcluster in der Gasphase erfolgen, die anderen oben genannten Thiole werden in Lösung innerhalb einer Reaktionszeit von etwa 12 h an Goldcluster angebunden.

Durch die Anbindungsmoleküle wird eine bessere Anbindung der anorganischen Nanocluster im bzw. am zweiten Substrat erzielt.

In einer besonders vorteilhaften Ausführungsform können als Anbindungsmoleküle Zellen verwendet werden. In diesem Fall binden die Adhäsionsproteine der Zelle abhängig von der Nanostruktur auf dem ersten Substrat nur an bestimmte Nanocluster. Dadurch kann erreicht werden, dass nach dem Abtrennen des ersten Substrats nur solche Nanocluster auf dem zweiten Substrat zurückbleiben, die an die Adhäsionsproteine der.Zelle gebunden sind. Auf diese Weise lassen sich wichtige Erkenntnisse über das Adhäsionsyerhalten von Zellen auf nanostrukturierten Oberflächen erhalten.

Gemäß einer weiteren bevorzugten Ausführungsform kann im Anschluss an den Schritt (iii) bzw. (d) eine Passivierung der die Nanocluster umgebenden Bereiche durchgeführt werden, um eine gezielte Wechselwirkung von z.B. Zellen oder anderen Biomolekülen mit den anorganischen Nanoclustern zu erzielen. Eine solche Passivierung durch Polyethylenglykol ist beispielsweise in der US-Patentanmeldung 2003/0133963 A1 von J.A. Hubbell beschrieben. Eine weitere Möglichkeit zur Passivierung besteht in der Erzeugung von Hydroxylgruppen auf den Oberflächen von Polystyrol- oder PDMS-Substraten durch ein Sauerstoff-Plasma. Auf diesen Hydroxylgruppen lassen sich dann durch Anbinden von (3-Triethoxysilyl-propyl)-carbamidsäure-(methoxypolyethylenglykol)-estern eine molekulare PEG-Monoschicht mit stark proteinabweisenden Eigenschaften herstellen. Außerdem ist bekannt, dass sich Polystyrol-Oberflächen im Sauerstoff-Plasma mit Bovin Serum Albumin (BSA) gegen Proteinwechselwirkungen und Zelladhäsion passivieren lassen. Um die unspezifische Anbindung von BSA an den Bereich der Struktur, der nicht passiviert werden soll, zu verhindern, muss dieser Bereich zuerst mit einer anderen spezifisch anbindenden Substanz geschützt werden. So können Goldcluster beispielsweise mit PEG-Thiolen geschützt werden, die nach einer Plasma-Aktivierung der Oberfläche angebunden werden. Die Bindung des Thiols zum Gold kann in Iod-Atmosphäre wieder zerstört werden. Nach der unspezifischen Anbindung des BSA an das Polystyrol kann das PEG dann abgewaschen werden (s. Promotionsarbeit Wolfgang Geyer, Universität Heidelberg, 04.05.2001).

Nach dem Schritt (iii) bzw. (d) der Abtrennung des ersten Substrats und ggf. nach der oben beschriebenen Passivierung kann ein Schritt der Biofunktionalisierung der anorganischen Nanocluster durchgeführt werden. "Biofunktionalisierung" bezeichnet einen Schritt, in dem spezifische Moleküle an die nanostrukturierten Cluster angebracht werden, um diese für bestimmte biologische Anwendungen zu funktionalisieren. Hierunter können alle Proteine, Proteinsequenzen und andere Moleküle, die von biologischem Interesse sind, verstanden werden. Diese Moleküle lassen sich direkt oder über verschiedene Anbindungsmoleküle an die Nanocluster anheften. Ein cyclisches RGD-Peptid mit Thiolanker lässt sich z.B. an die Goldcluster nach dem Passivierungsschritt anbinden. Die Goldcluster lassen sich so für die spezifische Anbindung von Integrinen, einem zellulären Adhäsionsprotein, funktionalisieren. Ein anderes Beispiel ist die Anbindung von molekularen Motoren wie Kinesin oder Myosin.

Gemäss einer weiteren bevorzugten Ausführungsform kann das im Schritt (b) aufgebrachte Substratmaterial vor oder nach der Härtung mit heterobifunktionalen Molekülen für die Anbindung weiterer Moleküle funktionalisiert werden. Das heterobifunktionale Molekül ist so aufgebaut, dass es mindestens zwei unterschiedliche funktionale Gruppen besitzt. Mindestens eine dieser funktionalen Gruppen ist derart, dass es an das härtbare Material des zweiten Substrats anbinden kann, während die chemische Funktionalität der anderen funktionalen Gruppe(n) auch nach der Anbindung erhalten bleibt. Hierfür eignen sich z.B. 2-Carboxyethylacrylat, Propenthiol und 2-Aminoethylmethacrylat. So kann zum Beispiel neben der Biofunktionalisierung der Nanostruktur eine Biofunktionalisierung der übrigen Substratoberfläche mit beliebigen Molekülen von biologischem Interesse vorgenommen werden. Von besonderem Interesse sind hierbei Wachstumsfaktoren wie EGF, NGF und TGF sowie Zelladhäsionsproteine bzw. Peptide wie Fibronectin, RGD und Catherine als auch extracellulare Signalmoleküle wie Vasopressin, Interferon, Insulin. Durch eine solche Funktionalisierung lassen sich Moleküle unterschiedlicher Art in geordneter Weise nebeneinander fixieren, so dass sie miteinander in geregelte Wechselwirkung treten. Beispielsweise lässt sich ein PEG Hydrogel, in das bei der Polymerisation Carboxylgruppen eingebunden werden, mit dem Wachstumsfaktor EGF aktivieren, während zusätzlich die Nanostrukturen wie oben beschrieben mit RGD für Zellanbindung funktionalisiert sind, was z.B. für Serum-freie Zellkulturen eingesetzt werden kann. Ein anderes Beispiel ist die geometrische Anordnung von viralen Spike-Proteinen an den Nanostrukturen mit einer Funktionalisierung des Hydrogels mit zellulären Wachstumsfaktoren.

Eine weitere vorteilhafte Ausführungsform stellt die Verwendung von dreidimensionalen ersten Substraten dar. Dieses dreidimensionale erste Substrat ist ganz oder teilweise auf der Oberfläche nanostrukturiert. In Schritt (b) wird dieses Substrat ganz oder teilweise in dem härtbaren Material eingebettet und in Schritt (d) herausgelöst, so dass eine auf der Oberfläche nanostrukturierte dreidimensionale Struktur erhalten wird. Das dreidimensionale erste Substrat kann eine beliebige dreidimensionale Form aufweisen, z.B. faser-, kugel- oder linsenförmig. Zum Beispiel kann das dreidimensionale erste Substrat eine Glasfaser mit einem Durchmesser von 5µm bis 300µm sein, deren Oberflächen durch das oben genannte Verfahren mit einer Nanostruktur aus Goldclustern beschichtet ist. Vorstellbar sind auch Kolloide, die mit Goldpunkten beschichtet werden. Die Goldpunkte werden durch die Immobilisierung von Cysteamin und Acryloylchlorid aktiviert und in PEG-DA eingegossen. Die Glassfasern können in Schritt (d) mit Flusssäure herausgelöst oder mechanisch abgelöst werden. Spezifische Aktivierung für die Anbindung bestimmter Zellen ermöglicht eine Verwendung als Nervenkanal oder Stent.

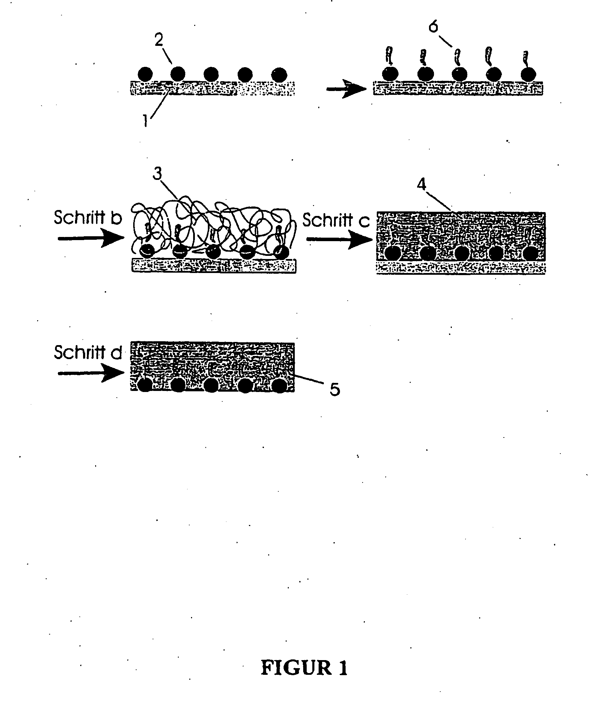

Das oben beschriebene Verfahren der vorliegenden Erfindung wird im folgenden unter Bezugnahme auf Figur 1 näher erläutert, wobei in Figur 1 das Verfahren zur Herstellung eines polymeren oberflächenstrukturierten Substrats der vorliegenden Erfindung gemäß einer bevorzugten Ausführungsform schematisch dargestellt ist.

Wie in Figur 1 gezeigt, werden zunächst in einem Schritt (a) anorganische Nanocluster 2 auf eine Oberfläche eines ersten Substrats 1 nanostrukturiert aufgebracht. Anschließend werden Anbindungsmoleküle 6 auf dieser Nanostruktur immobilisiert. In dem nachfolgenden Schritt (b) wird dann ein härtbares Substratmaterial 3 für ein zweites Substrat auf die nanostrukturierte Oberfläche des ersten Substrats 1 aufgebracht. Anschließend wir im Schritt (c) das härtbare Substratmaterial 3 für ein zweites Substrat gehärtet, wodurch das zweite Substrat 4 erhalten wird. In dem Schritt (d) wird das erste Substrat 1 dann von dem zweiten Substrat 4 und den Nanoclustern 2, einschließlich der Anbindungsmoleküle 6, abgetrennt, wodurch ein mit Nanoclustern nanostrukturiertes zweites Substrat 5 erhalten wird.

Gemäß einer alternativen Ausführungsform wird die obige Aufgabe der Erfindung auch gelöst durch ein Verfahren zur Herstellung eines polymeren oberflächenstrukturierten Substrats, umfassend die Schritte:
(a') Aufnehmen eines Polymers in einem geeigneten Lösungsmittel unter Bildung eines gelösten Kern-Schale-Polymersystems, -
(b') Beladen von mindestens einem Teil der Kerne des Kern-Schale-Polymersystem mit einer oder mehreren, gleichen oder unterschiedlichen Metallverbindungen, die Nanocluster bilden,
(c') Aufbringen des in Schritt (b') erhaltenen Kern-Schale-Polymersystems als Film derart auf mindestens eine Seite eines Substrats, dass das Kern-Schale-Polymersystem in einer regelmäßigen Struktur im Film angeordnet ist, und
(d') Teilweises Entfernen des in Schritt (c') auf dem Substrat aufgebrachten Polymers des Kern-Schale-Polymersystems, wodurch die Nanocluster nicht mehr vollständig von dem Polymer umgeben sind, und
(e') Abtrennen des Substrats von dem in Schritt (d') erhaltenen Film, wodurch ein mit Nanoclustern nanostrukturierter polymerer Film erhalten wird.

Die Schritte (a') bis (c') entsprechen der Vergehensweise, die üblicherweise in dem mizellaren Blockcopolymer-Nanolithographie-Verfahren angewendet werden. Details zur Durchführung dieser Schritte finden sich in den Druckschriften DE 199 52 018, DE 197 47 813, DE 297 47 815 bzw. DE 197 47 816.

Unter dem Ausdruck "Kern-Schale-Polymersystem" sind beispielsweise makromolekulare Amphiphile zu verstehen, welche in wässriger oder organischer Lösung assoziieren und wohldefinierte kugelförmige bzw. stäbchenförmige Mizellen, Lamellen, Vesikel oder komplexe Aggregate bilden können. Erfindungsgemäß sind damit auch solche, allgemein als Wirt/Gast-Systeme bezeichnete Systeme eingeschlossen, in denen ein von dem eingesetzten Polymer (Wirtsverbindung) erzeugter Molekülhohlraum bzw. Molekülinnenraum, d. h. der Polymerkern, mit einer Gastverbindung, d.h. der verwendeten Metallverbindung, beladen bzw. komplexiert werden kann.

Das erfindungsgemäß eingesetzte Polymer, welches das Kern-Schale-Polymersystem aufbaut, ist vorzugsweise aus Blockcopolymeren, Pfropfcopolymeren, Mikroarmsternpolymeren, Sternpolymeren mit unterschiedlichen Armen, dentritischen Polymeren, Mikrogelteilchen, Sterriblockpolymeren, Blocksternpolymeren und Kern-Schale-Latexpolymeren ausgewählt.

Mehr bevorzugt ist das Polymer Polystyrol-b-polyethylenoxid, Polystyrol-poly(2-vinylpyridin), Polystyrol-poly(4-vinylpyridin) oder ein Gemisch davon. Der Polystyrolblock darin kann aber auch durch andere nicht-polare Polymere, wie beispielsweise Polyisopren, Polybutadien, Polymethylmethacrylat oder andere Polymethacrylate ersetzt werden. Der zweite bzw. polare Block in einem solchen Zweiblockcopolymer kann ein solcher sein, der eine möglichst starke Wechselwirkung mit der eingesetzten Metallverbindung eingeht. Beispiele hiefür.sind Polyacrylsäure, Polymethacrylsäure, aminosubstituierte Polystyrole, Polyacrylate bzw. Polymethacrylate, aminosubstituierte Polydiene, Polyethylenimine, verseifte Polyoxazoline oder hydriertes Polyacrylnitril. Der erste Block kann auch aus einem polaren Polymer aufgebaut sein, jedoch mit der Maßgabe, dass dann die Metallverbindung derart gewählt ist, dass diese hauptsächlich, d.h. selektiv, mit dem zweiten polaren Block wechselwirkt.

Typischerweise werden die vorgenannten Polymersysteme in einem selektiven Lösungsmittel, wie z. B. Toluol, in einer Menge von etwa 10³ bis 100 mg/ml, vorzugsweise etwa 5 mg/ml, gelöst. Nach etwa 12 Stunden wird in Schritt (b') des erfindungsgemäßen Verfahrens die Lösung mit einer oder mehreren Metallverbindungen versetzt und für 24 Stunden stark gerührt, um mindestens einen Teil der durch das Kern-Schale-Polymersystem gebildeten Polymerkerne mit der/den Metallverbindung(en) zu beladen.

Die Metallverbindungen sind dieselben wie die für die oben beschriebenen Metallcluster. Bevorzugt ist auch in dieser alternativen Ausführungsform Au bzw. eine Au-Verbindung.

In Schritt (c') des erfindungsgemäßen Verfahrens wird das Aufbringen des Films in Mono- oder Multischichten auf mindestens eine Seite eines Substrats vorzugsweise durch Tauch-, Gieß-, Spinschleuderverfahren oder durch Adsorption aus verdünnter Lösung durchgeführt. Mehr bevorzugt wird das Aufbringen in Mono- oder Multischichten durch Tauchverfahren in verdünnter Lösung durchgeführt. In einer bevorzugten Ausführungsform wird bzw. werden vor Schritt (c') die im Polymerkern enthaltende(n) Metallverbindung(en) durch chemische Behandlung und/oder durch energiereiche Strahlung, beispielsweise UV-Licht, Röntgenstrahlung oder Elektronenbeschuss, in Lösung oder im Film in das Metall oder ein Metalloxid überführt.

Als verwendbare Substratmaterialien für das Substrat im Fall des mizellaren Blockcopolymer-Nanolithographie-Verfahrens sind insbesondere zu nennen: Edelmetalle, oxidische Gläser, mono- oder multikristalline Substrate, Halbleiter, Metalle mit oder ohne passivierter Oberfläche, Isolatoren oder allgemein Substrate mit hoher Resistenz gegen nachfolgende Ätzprozeduren. Vorzugsweise handelt es sich hierbei um Pt, Au, GaAs, AlxGaAs, Si, Si02, Ge, SixNy, SixGaAs, InP, InPSi, GaInAsP, Glas, Graphit, Diamant, Glimmer, SrTiO3 sowie deren dotierte Modifikationen.

Die in Schritt (c') erhaltenen Filme, d.h. makroskopisch deckende Filme, werden beispielsweise durch definiertes Ziehen eines Substrats aus der Lösung mit Geschwindigkeiten zwischen beispielsweise 0,001 mm/min und 2 m/min erzielt. Die mit der Metallverbindung beladenen Polymerkerne werden dabei unter Ausbildung einer regelmäßigen Struktur im Film im wesentlichen intakt abgeschieden.

In Schritt (d') des erfindungsgemäßen Verfahrens wird der Film zusammen mit dem vom Film mindestens teilweise bedeckten Substrat einem reaktiven Ionenätzverfahren, einem Ionensputterverfahren oder einem nasschemischen Verfahren oder einer Kombination davon unterworfen. Die auf der Substratoberfläche abgeschiedenen Strukturen dienen dabei als Maske, die durch Ätztechniken in das entsprechende Substrat übertragen werden, wobei nur ein Teil des auf dem Substrat aufgebrachten Films rückstandsfrei an der gewünschten Stelle bzw. dem gewünschten Bereich entfernt wird, und worin die durch das Kern-Schale-Polymersystem erzeugte, regelmäßige Struktur aufgrund und in Abhängigkeit von der Art der Beladung.der Polymerkerne sowie der Dauer des reaktiven Ionenätzverfahrens und/oder des Ionensputterverfahrens und/oder des nasschemischen Verfahrens in eine Reliefstruktur des Substrats überführt wird. Vorzugsweise wird ein Ionenätzen mit Argon, Ozon, Sauerstoff und deren Mischungen durchgeführt, mehr bevorzugt ist ein Argon-Ionen-Sputtern. Der Ausdruck "teilweises Entfernen" bedeutet, dass die Nanocluster nach dem teilweisen Entfernen des Polymers nicht mehr vollständig von dem Polymer umgeben sind. Bevorzugt bleiben 20 bis 80 % der Oberfläche der anorganischen Cluster mit dem Polymer umgeben. Bevorzugter ist ein Bereich von 30 bis 70 % der Oberfläche der Cluster nach dem teilweisen Entfernen des aufgebrachten Films mit Polymer bedeckt, noch bevorzugter 40 bis 60 %, und am meisten bevorzugt 50 %.

Die Dicke des in Schritt (c') aufgebrachten Films ist bevorzugt 1 nm bis einige cm, insbesondere 10 nm bis 100 cm, besonders bevorzugt 10 nm bis 1 cm, noch bevorzugter 100 nm bis 1 mm.

Der Schritt (e') des Abtrennens des Substrats von dem in Schritt (d') erhaltenen Films erfolgt auf die gleiche Weise wie für Schritt (d) gemäß Anspruch 1 beschrieben.

Die aus den oben beschriebenen Verfahren gemäß der vorliegenden Anmeldung erhältlichen polymeren Substrate mit nanostrukturierten Oberflächen können insbesondere auf Implantat- und Stentmaterialien aufgebracht und als Kultursubstrate für Zellen, Bakterien und Viren, als Nährböden für Differenzierungsexperimente von Stammzellen und Nährböden für Gewebe verwendet werden. Weiche polymere Substrate spielen insbesondere für neuronales Wachstum eine Rolle. Außerdem können sie auch für elektronische Bauteile eingesetzt und zur Benetzung/Entnetzung und gegen Verschmutzung von Gegenständen verwendet werden. Ein Beispiel dafür ist die Anwendung in einer Serum-freien Zellkultur für die Erzeugung von Hautersatz. Darüber hinaus sind durch das erfindungsgemäße Verfahren erhältlichen Substrate mit nanostrukturierten Oberflächen wichtig für unterschiedlichste Anwendungen, bei denen über eine genaue Anordnung von biologisch aktiven Molekülen biologische Systeme imitiert, manipuliert, untersucht und quantifiziert werden, z.B. Imitation von Virus- oder Pollen-Wechselwirkungen mit Zellen, Manipulation der Zelldifferenzierung zu erwünschten Phenotypen und die Anbindung molekularer Motoren zur Untersuchung und Quantifizierung ihrer Aktivität. Außerdem können sie auch für elektronische und optische Bauteile, wobei hier die Verwendung von halbleitenden Substraten von besonderem Interesse ist, sowie als biologische oder chemische Sensoren eingesetzt und zur Benetzung/Entnetzung und gegen Verschmutzung von Gegenständen verwendet werden.

Ein besonderer Vorteil der vorliegenden Erfindung ist weiterhin, dass über die Geometrie und chemische und physikalische Beschaffenheit der Oberfläche, auf der eine Zelle adhärieren kann, die Bindungsrezeptoren und die Proteine in der Zelle auf eine Weise angeordnet und positioniert werden können, dass die Funktion der Zelle, z.B. Aktivität von biochemischen Signalwegen, Genexpression und die Synthese von bestimmten zellulären Proteinen, darüber gezielt gesteuert werden können.

Im Folgenden wird die vorliegende Erfindung durch Beispiele näher erläutert.

### Beispiel 1

### Übertragung von Goldstrukturen von einer Glasoberfläche auf Polystyrol

Ein Exsikkator mit dem Glassubstrat mit der zu übertragenden Goldstruktur wird evakuiert und mit einem Schlenkkolben verbunden, in welchem sich einige µl Propenthiol befinden. Das so verdampfte Propenthiol wirkt für 12 Stunden auf die Substratoberfläche ein.
Nach Belüften und Spülen des Substrats im Stickstoffgegenstrom wird eine Polystyrol/Toluol-Lösung (25 mg/ml) durch einen 0,2 µm Spritzenfilter auf die Substratoberfläche aufgetropft (ca. 5µl/cm²).
Nach 6 Stunden Trockenzeit bei Raumtemperatur wird das Polystyrol für 1 Stunde bei 60 °C im Ofen ausgehärtet.
Das Substrat wird mit der Polystyrol bedeckten Seite nach oben in eine 12%ige Flusssäure-Lösung gelegt. Nach einigen Sekunden schwimmt der Polystyrolfilm auf, wird mit MQ-Wasser gespült und mit Stickstoff trockengeblasen.

### Beispiel 2

### Übertragung von Goldstrukturen von einer Glasoberfläche auf Polyethylenglykol

Das Glassubstrat mit den zu übertragenden Goldstrukturen wird für 12 Stunden in eine 5%ige Lösung aus PEG-dithiol in DMF gelegt.
Nach Spülen in MQ-Wasser wird im Stickstoffgegenstrom eine Lösung aus 300 mg PEG-Diacrylat (PEG-DA), 0,15 mg Photoinitiator (Irgacure 2959) und 300ml Wasser aufgetragen (ca. 5µl/cm²).
Unter Stickstoff wird für 45 min mit einer UV Lampe (275nm) bestrahlt.
Das Substrat wird mit der Glasseite nach oben in einer Weise mit 12%iger Flussäurelösung betropft, dass keine Flusssäure über den Rand des Glases hinweg mit dem PEG-Hydrogel in Kontakt kommt. Nach einigen Stunden löst sich das Glas von dem Hydrogelfilm.

Das Hydrogel wird mehrfach in MQ-Wasser gewaschen und im Wasser aufbewahrt.

### Beispiel 3

### Übertragung von Goldstrukturen in eine PEG Röhre

Die mit Gold-Nanostrukturen strukturierte Glasfaser mit einem Durchmesser von 50 - 150 µm für 3 Stunden in Propenthiol-Dampf hängen. Die Faser wird anschließend in einen Tropfen PEG-DA mit Photoinitiator Irgacure 2959 gelegt. Nach 45 minütiger UV Bestrahlung unter Stickstoff wird die Glasfaser mit einem Ende in einer Weise in eine 12%ige Flussäurelösung gehalten, dass der verfestigte PEG Tropfen noch zu ca. zwei Dritteln aus der Säure ragt. Sobald die Flusssäure, die entlang der Glasfaser im Inneren des PEG Tropfens nach oben steigt, das obere Ende des PEG Tropfens erreicht, ist die Faser in ihrem Durchmesser so dezimiert, dass sie aus dem PEG Tropfen herausgezogen werden kann und die Goldstrukturen im Inneren der entstandenen Röhre zurück lässt.

### Beispiel 4

### Herstellung einer biofunktionalisierten PEG Matrix mit Gold Nanostrukturen

1 mg 2-Carboxyethylacrylat wird in 500 ml PEG-DA 700 gelöst. Die Lösung wird gemäss Beispiel 2 zur Übertragung von Goldstrukturen eingesetzt. Nach der Polymerisation und dem Übertrag der Goldstrukturen auf das PEG lassen sich die Carboxyl Gruppen in N-Hydroxy-succinimid-ester überführen. Dies geschieht durch Überführen der Substrate in eine 1%ige Lösung aus N-Hydroxysuccinimide in einem HEPES Puffer (pH 7,3). Eine darauf folgende Anbindung von Tris-(carboxymethyl)-amin (NTA) in getrocknetem DMSO und die Komplexierung mit Nickel ermöglicht die Anbindung beliebiger Proteine über Histidin. Nach der Anbindung von NTA und vor der Nickel-Komplexierung können die Goldstrukturen über eine Thiolgruppe mit einem bioaktiven Molekül z.B. RGD funktionalisiert werden.

## Patentansprüche

1. Verfahren zur Herstellung eines oberflächenstrukturierten Substrats, umfassend die Schritte:
(i) Herstellen eines ersten Substrats, das auf mindestens einer Oberfläche mit anorganischen Nanoclustern nanostrukturiert ist,
(ii) Aufbringen eines Substratmaterials für ein zweites substrat, das vom ersten Substratmaterial verschieden ist, auf die im Schritt (i) erhaltene nanostrukturierte Oberfläche des ersten Substrats, und
(iii) Abtrennen des ersten Substrats von dem zweiten Substrat aus Schritt (ii) und den anorganischen Nanoclustern, wodurch ein mit Nanoclustern nanostrukturiertes zweites Substrat erhalten wird.

2. Verfahren gemäß Anspruch 1, wobei die Aufbringung des zweiten Substratmaterials in Schritt (ii) durch thermische Verdampfung, Elektronenstrahlverdampfung, Sputtern oder elektrochemisches Abscheiden erfolgt.

3. Verfahren gemäß Anspruch 2, wobei das zweite Substratmatarial ein Leiter oder Halbleiter ist, ausgewählt aus Si, C, Zinkoxid, Cr, Indiumoxid, Cu, Hexadecafluorphthalocyanin (F16CuPc), Indiumarsenid und Galliumarsenid, oder Aluminiumoxid, Calciumfluorid oder Magnesiumfluorid ist,

4. Verfahren zur Herstellung eines polymeren oberflächenstrukturierten Substrats, umfassend die Schritte:
(a) Herstellen eines ersten Substrats (1), das auf mindestens einer Oberfläche mit anorganischen Nanoclustern (2) nanostrukturiert ist,
(b) Aufbringen eines härtbaren Substratmaterials (3) für ein zweites Substrat, das vom ersten Substratmaterial verschieden ist, auf die im Schritt (a) erhaltene nanostrukturierte Oberfläche des ersten Substrats, wobei das härtbare Substratmaterial ausgewählt ist aus einem organischen vernetzbaren oder nichtvernetzbaren Polymer, einem Harz, einem organischen polymerisierbaren und/oder vernetzbaren oligomer und einem organischen polymerisierbaren Polymerprecursor oder Mischungen davon,
(c) Härten des Substratmaterials für das zweite Substrat und
(d) Abtrennen des ersten Substrats von dem in Schritt (c) erhaltenen zweiten Substrat (4) und den anorganischen Nanoclustern, wodurch ein mit Nanoclustern nanostrukturiertes zweites Substrat (5) erhalten wird.

5. Verfahren gemäß Anspruch 4, worin zwischen Schritt (a) und Schritt (b) ein Schritt der Immobilisierung eines Anbindungsmoleküls (6) durchgeführt wird.

6. Verfahren gemäß Anspruch 5, worin das Anbindungsmolekül ausgewählt ist aus Propenthiol, Mercaptopolyethylenglykolacrylat, Polyethylenglykoldithiol, Cysteamin mit Acryloylchlorid, Alkylthioglykolat und Amino-1-alkylthiol, worin Alkyl einen geraden oder verzweigten, gesättigten oder ungesättigten Kohlenwasserstoff mit 1 bis 24 Kohlenstoffatomen bezeichnet.

7. Verfahren gemäß Anspruch 5, worin die Anbindungsmoleküle Rezeptoren einer Zelle sind.

8. Verfahren zur Herstellung eines polymeren oberflächenstrukturierten Substrats, umfassend die Schritte:
(a') Aufnehmen eines Polymers in einem geeigneten Lösungsmittel unter Bildung eines gelösten Kern-Schale-Polymersystems,
(b') Beladen von mindestens einem Teil der Kerne des Kern-Schale-Polymersystem mit einer oder mehreren, gleichen oder unterschiedlichen Metallverbindungen, die Nanocluster bilden,
(c') Aufbringen des in Schritt (b) erhaltenen Kern-Schale-Polymersystems als Film derart auf mindestens eine Seite eines Substrats, dass das Kern-Schale-Polymersystem in einer regelmäßigen Struktur im Film angeordnet ist,
(d') teilweises Entfernen des in Schritt (c') auf dem Substrat aufgebrachten Polymers des Kern-Schale-Polymersystems, wodurch die Nanocluster nicht mehr vollständig von dem Polymer umgeben sind, und
(e') Abtrennen des Substrats von dem in Schritt (d') erhaltenen Film, wodurch ein mit Nanoclustern nanostrukturierter polymerer Film erhalten wird.

9. Verfahren gemäß einem der Ansprüche 4 bis 8, worin das erste Substrat aus Schritt (i) oder Schritt (a) eine dreidimensionale Form besitzt.

10. Verfahren gemäß einem der Ansprüche 1 bis 9, worin die anorganischen Cluster Au-Cluster sind.

11. Verfahren gemäß einem der Ansprüche 1 bis 10, worin die anorganischen Nanocluster mit einem Abstand von 1 nm bis 300 µm strukturiert angeordnet sind.

12. Substrat mit strukturierter Oberfläche, erhältlich durch ein Verfahren gemäß Anspruch 4 oder 8.

13. Verwendung des Substrats gemäß Anspruch 12 zur Aufbringung auf ein Stent- oder Implantatmaterial.

14. Verwendung des Substrats gemäß Anspruch 12 zur Adhäsion von Zellen, Viren und/oder Bakterien.

15. Verwendung des Substrats gemäß Anspruch 12 zur strukturierten Anbindung biologisch aktiver Moleküle zur Imitierung, Manipulation, Untersuchung und Quantifizierung biologischer Systeme.

16. Verwendung des polymeren Substrats gemäß Anspruch 12 für die Herstellung elektronischer und optischer Bauteile und chemischer Sensoren.

## Claims

1. A method for the production of a surface-structured substrate, comprising the steps:
(i) production of a first substrate that is nanostructured on at least one surface with inorganic nanoclusters,
(ii) application of a substrate material for a second substrate different from the first substrate material on the nanostructured surface of the first substrate, which surface was obtained in step (i), and
(iii)separation of the first substrate from the second substrate from step (ii), including the inorganic nanoclusters, as a result of which a second substrate nanostructured with nanoclusters is obtained.

2. The method according to Claim 1, wherein the application of the second substrate material takes place in step (ii) by thermal evaporation, electron beam evaporation, sputtering or electrochemical deposition.

3. The method according to Claim 2, wherein the second substrate material is a conductor or semiconductor selected from Si, C, zinc oxide, Cr, indium oxide, Cu, hexadecafluorophthalocyanin (F16CuPc), indium arsenide and gallium arsenide or aluminum oxide, calcium fluoride or magnesium fluoride.

4. A method for the production of a polymeric surface-structured substrate, comprising the steps:
(a) production of a first substrate (1) that is nanostructured on at least one surface with inorganic nanoclusters (2),
(b) application of a hardenable substrate material (3) for a second substrate different from the first substrate material on the nanostructured surface of the first substrate, which surface was obtained in step (a), the hardenable substrate material being selected from an organic cross-linkable or non-cross-linkable polymer, a resin, an organic polymerizable and/or cross-linkable oligomer and an organic polymerizable polymer precursor or mixtures thereof,
(c) hardening of the substrate material for the second substrate and
(d) separation of the first substrate from the second substrate (4) obtained in step (c) and the inorganic nanoclusters, as a result of which a second substrate (5) nanostructured with nanoclusters is obtained.

5. The method according to Claim 4, in which a step of immobilizing a binding molecule (6) is carried out between step (a) and step (b).

6. The method according to Claim 5, in which the binding molecule is selected from propenethiol, mercaptopolyethyleneglycolacrylate, polyethyleneglycoldithiol, cysteamine with acryloylchloride, alkylthioglycolate and amino-1-alkylthiol, wherein alkyl designates a straight or branched, saturated or unsaturated hydrocarbon with 1 to 24 carbon atoms.

7. The method according to Claim 5, in which the binding molecules are receptors of a cell.

8. A method for the production of a polymeric surface-structured substrate, comprising the steps:
(a') taking up a polymer in a suitable solvent under formation of a dissolved core shell polymer system,
(b') charging of at least one part of the cores of the core shell polymer system with one or more, same or different metal compounds that form nanoclusters,
(c') application of the core shell polymer system obtained in step (b') as a film onto at least one side of a substrate in such a manner that the core shell polymer system is arranged in a regular structure in the film,
(d') partial removal of the polymer of the core shell polymer system applied on the substrate in step (c'), as a result of which the nanoclusters are no longer completely surrounded by the polymer, and
(e') separation of the substrate from the film obtained in step (d'), as a result of which a polymeric film nanostructured with nanoclusters is obtained.

9. The method according to any one of Claims 4 to 8, in which the first substrate from step (i) or step (a) has a three-dimensional form.

10. The method according to any one of Claims 1 to 9, in which the inorganic clusters are Au clusters.

11. The method according to any one of Claims 1 to 10, in which the inorganic nanoclusters are arranged in a structured manner with a distance of 1 nm to 300 µm.

12. A substrate with a structured surface, obtainable by a method according to Claim 4 or 8.

13. The use of the substrate according to Claim 12 for the application on a stent material or implant material.

14. The use of the substrate according to Claim 12 for the adhesion of cells, viruses and/or bacteria.

15. The use of the substrate according to Claim 12 for the structured binding of biologically active molecules for the imitation, manipulation, examination and quantification of biological systems.

16. The use of the polymeric substrate according to Claim 12 for the production of electronic and optical components and chemical sensors.

## Revendications

1. Procédé de fabrication d'un substrat structuré en surface, comprenant les étapes consistant à :
(i) fabriquer un premier substrat, qui est nanostructuré avec des nanoclusters inorganiques sur au moins une surface,
(ii) déposer une matière de substrat pour un deuxième substrat, qui est différente de la première matière de substrat, sur la surface nanostructurée du premier substrat, obtenue à l'étape (i), et
(iii) séparer le premier substrat du deuxième substrat de l'étape (ii) et des nanoclusters inorganiques, ce qui permet d'obtenir un deuxième substrat nanostructuré avec des nanoclusters.

2. Procédé selon la revendication 1, dans lequel le dépôt de la deuxième matière de substrat à l'étape (ii) s'effectue par vaporisation thermique, vaporisation par jet électronique, pulvérisation ou dépôt électrochimique.

3. Procédé selon la revendication 2, dans lequel la deuxième matière de substrat est un conducteur ou un semiconducteur, choisi parmi Si, C, oxyde de zinc, Cr, oxyde d'indium, Cu, hexadécafluorophtalocyanine (F16CuPc), arséniure d'indium et arséniure de gallium, ou oxyde d'aluminium, fluorure de calcium ou fluorure de magnésium.

4. Procédé de fabrication d'un substrat polymère structuré en surface, comprenant les étapes consistant à :
(a) fabriquer un premier substrat (1), qui est nanostructuré avec des nanoclusters (2) inorganiques sur au moins une surface,
(b) déposer une matière de substrat (3) durcissable pour un deuxième substrat, qui est différente de la première matière de substrat, sur la surface nanostructurée du premier substrat, obtenue à l'étape (a), où la matière de substrat durcissable est choisie entre un polymère organique réticulable ou non réticulable, une résine, un oligomère organique polymérisable et / ou réticulable et un précurseur de polymère organique polymérisable ou des mélanges de ceux-ci,
(c) durcir la matière de substrat pour le deuxième substrat et
(d) séparer le premier substrat du deuxième substrat (4), obtenu à l'étape (c), et des nanoclusters inorganiques, ce qui permet d'obtenir un deuxième substrat (5) nanostructuré avec des nanoclusters.

5. Procédé selon la revendication 4, dans lequel une étape d'immobilisation d'une molécule de liaison (6) est réalisée entre l'étape (a) et l'étape (b).

6. Procédé selon la revendication 5, dans lequel la molécule de liaison est choisie parmi le propènethiol, le mercaptoacrylate de polyéthylène glycol, le dithiol de polyéthylène glycol, la cystéamine avec du chlorure d'acryloyle, l'alkylthioglycolate et l'amino-1-alkylthiol, où alkyle désigne un hydrocarbure linéaire ou ramifié, saturé ou insaturé ayant de 1 à 24 atomes de carbone.

7. Procédé selon la revendication 5, dans lequel les molécules de liaison sont des récepteurs d'une cellule.

8. Procédé de fabrication d'un substrat polymère structuré en surface, comprenant les étapes consistant à :
(a') diluer un polymère dans un solvant approprié en formant un système polymère noyau - enveloppe,
(b') charger au moins une partie des noyaux du système polymère noyau - enveloppe avec un ou plusieurs composés métalliques identiques ou différents, qui forment des nanoclusters,
(c') déposer le système polymère noyau - enveloppe obtenu à l'étape (b) sous forme de film sur au moins une face d'un substrat de sorte que le système polymère noyau - enveloppe soit disposé en une structure régulière dans le film,
(d') retirer partiellement le polymère du système polymère noyau - enveloppe, déposé sur le substrat à l'étape (c'), moyennant quoi les nanoclusters ne sont plus entièrement entourés par le polymère, et
(e') séparer le substrat du film obtenu à l'étape (d'), ce qui permet d'obtenir un film polymère nanostructuré avec des nanoclusters.

9. Procédé selon l'une des revendications 4 à 8, dans lequel le premier substrat de l'étape (i) ou de l'étape (a) possède une forme tridimensionnelle.

10. Procédé selon l'une des revendications 1 à 9, dans lequel les clusters inorganiques sont des clusters Au.

11. Procédé selon l'une des revendications 1 à 10, dans lequel les nanoclusters inorganiques sont disposés, structurés, à une distance de 1 nm à 300 µm.

12. Substrat avec une surface structurée, pouvant être obtenu par un procédé selon la revendication 4 ou 8.

13. Utilisation du substrat selon la revendication 12 pour le dépôt sur un matériau d'endoprothèse ou d'implant.

14. Utilisation du substrat selon la revendication 12 pour l'adhésion de cellules, de virus et / ou de bactéries.

15. Utilisation du substrat selon la revendication 12 destinée à la liaison structurée de molécules biologiquement actives pour l'imitation, la manipulation, l'analyse et la quantification de systèmes biologiques.

16. Utilisation du substrat polymère selon la revendication 12 pour la fabrication de composants électroniques et optiques et de capteurs chimiques.
